# EUROPEAN PATENT SPECIFICATION

(11) **EP 0 570 712 B1**
(45) Date of publication and mention of the grant of the patent: **22.07.1998**
(21) Application number: 93106508.0
(22) Date of filing: 21.04.1993
(51) Int. Cl.: A61N 1/05

(54) **Electrode arrangement**
Elektrodenanordnung
Arrangement d'électrodes

(30) Priority: 21.05.1992 SE 9201600
(43) Date of publication of application: 24.11.1993
(73) Proprietor: Pacesetter AB, 175 84 Järfälla (SE)
(72) Inventor: Hirschberg, Jakub, S-183 44 Täby (SE); Neubauer, Heinz, S-175 70 Järfälla (SE); Gilljam, Nina, S-123 44 Farsta (SE); Lindegren, Ulf, S-122 35 Enskede (SE); Bowald, Staffan, S-740 10 Almunge (SE)
(74) Representative: Lettström, Richard Wilhelm

(56) References cited:
- US-A- 4 357 946
- US-A- 4 463 765
- US-A- 4 972 847
- US-A- 5 016 645

## Description

The invention relates to an electrode device for a medical apparatus of the type mentioned in the preamble of claim 1.

Such an electrode device is known from US-A 4 357 946 in which the rotation of a stylet causes a helical fixation screw to be advanced at a right-angle out of a thick fixation pad.

Another such electrode device is known from US-A-4 355 642. This document describes a cardiac electrode device comprising an electrode with an electrically conductive webbing constituting an electrode surface and a fixed helical with a pointed tip, said helical mounted on the electrode at a right angle to the electrode surface. When the electrode is to be affixed to the heart, the helical is screwed into the epicardial tissue by rotation of the entire electrode until the electrode's surface comes into contact with the tissue.

To give a physician access to screw the prior art electrode into place, the patient must undergo thorax surgery in which the chest is opened to expose the heart. The electrode can only be affixed to the exposed part of the heart. Screwing into the heart is traumatic to heart tissue and leads to the formation of scar tissue in the cardiac epicardium. Moreover, the electrode must be correctly situated at the initial screwing-in, since it cannot be subsequently moved without damage to the heart.

Since it is relatively thick in relation to its area, the electrode is stiff and, therefore, unable to assume the large area enabling transmission of a pulse to the largest possible part of the heart. Since the electrode is screwed in by being rotated until the electrode surface comes into contact with heart tissue, the electrode's orientation relative to heart tissue is governed by the screwing-in. The electrode must therefore have a rotationally symmetrical surface.

US-A-3,737,579 describes an epicardial pace electrode device comprising an electrode catheter, a helical electrode mounted at a right angle to the electrode catheter's longitudinal axis and Dacron Trade mark webbing. With the aid of a special, essentially tubular tool into which the pace electrode device is inserted in such a way that the helical points in the direction of the tubular tool's longitudinal axis, the helical can be screwed into the heart when the tool is rotated until the Dacron webbing comes into contact with heart tissue. The electrode can then be additionally affixed if the Dacron webbing is sutured to the heart.

In this instance, the electrode area is small, since a pace electrode is involved, but the electrode area could not be much larger, since it is screwed at a right angle into heart tissue. As a result of the fixed helical in the abovementioned prior art cardiac electrode device, electrode fixation and orientation in respect to the heart are performed simultaneously. Therefore, orientation of the electrode's placement arises from fixation, so the electrode's position after the screwing-in cannot be influenced. Here, this means that the electrode catheter could end up pointed up or down after implantation.

EP-B-0 211 166 describes a defibrillator electrode device containing a plurality of electrodes. Each electrode comprises a fixation plate with a screw for affixing the electrode to the heart. The screw points at a right angle to the electrode catheter's longitudinal axis. Around the fixation plate, there are a plurality of electrode helicals which form the active electrode surface. At implantation, a tool is used which is similar to the one described above in conjunction with the prior art pace electrode device (US-A-3,737,579). The tool is connected to the fixation plate so the screw becomes parallel to the tool's longitudinal axis. The electrode helicals are raised from the fixation plate along the tool and held in position with needles. When the tool is rotated, the electrode is screwed into heart tissue until the fixation plate comes into contact with the heart, whereupon the electrode helicals are detached from the tool, applied to the heart tissue and affixed to same with the needles. When all the electrodes have been attached to the heart, they are interconnected into a common electrode catheter.

A plurality of electrodes is used to cover a large part of heart tissue, making more comprehensive implantation surgery necessary. The plurality of electrodes also inflicts a large number of lesions on heart tissue, thereby increasing the risk of post-operative complications. The electrodes must be symmetrical for the same reason noted above for the prior art electrode devices. The fixation plate is stiff, and the direction of the electrode catheters in the installed position cannot be determined in advance, thereby increasing the difficulty in interconnecting them.

For endocardial electrode devices, the use of movable helicals for attaching an electrode to heart tissue is prior art. One such device is described in US-A-4,311,153. During introduction into the heart, the helical is enclosed in a sleeve constituting part of the electrode catheter so no tissue is damaged. The shell is coated with a membrane to prevent the entry of body fluids. Inside the heart, the helical is advanced to affix the electrode to endocardial tissue. The helical is advanced in a direction parallel to the electrode catheter by rotation of a connection contact attached to the helical or with a detachable stylet inside the electrode catheter.

Another prior art endocardial electrode device is described in US-A-4,922,927 and contains a defibrillation electrode extending the length of an electrode catheter and a pace electrode at the end of the electrode catheter. A moveable helical is attached to the same end. In the implantation of the electrode device, the helical can be manipulated with a stylet so it rotates around an axis parallel to the electrode catheter and can thereby be screwed into heart tissue for fixation of the electrode device. The electrode device can be removed from tissue if the screw is turned in the opposite direction.

As already noted, the axis of rotation of the helical in both the prior art endocardial electrode devices is parallel to the electrode catheter's axis, so the electrode devices can be pressed against heart tissue during implantation when the helical is screwed in, thereby guaranteeing firm seating of the helical even though the helical is remotely manipulated. The corresponding effect is obtained for the epicardial electrode devices in which a tool is employed for aiming the helical at heart tissue and for rotating the entire electrode.

The object of the invention is to produce an electrode device, as described above, which, without extensive surgery, can be implanted, remotely manipulated to minimize surgery and repositioned a plurality of times during the operation.

This is achieved in accordance with the invention in that the electrode device, as described in the preamble, is devised so the electrode is thin and flexible, the electrode extends substantially in a single plane, and the electrode surface is spread out in said single plane.

The thin, flexible electrode conforms with the contours of the tissue and can, thanks to the movable fixation element, even be devised with a large electrode surface conforming to tissue contours, since electrode placement and orientation is a separate operation and electrode fixation to tissue is another separate operation. The fixation element can be easily advanced with the stylet to affix the electrode or withdraw if relocation or a position adjustment is necessary. As a result of its flexibility, the electrode can also be coiled and advanced against tissue, e.g. through an introducer catheter, thereby reducing the necessary scope of surgery. The electrode can be moved with the stylet until it reaches the desired position and can be affixed. Since this operation is controlled from outside the patient receiving the surgery, the necessary scope of surgery is reduced even further.

As previously noted, the two prior art movable helicals (US-A-4,311,153, US-A-4,922,927) are advanced parallel to the electrode catheter. This would not be possible with the other prior art electrode devices (US-A-4,355,642, EP-B-0 211 166) which have a stiff electrode surface which is also parallel to the electrode catheter. The electrode surface would then not come into contact with tissue. Making the fixed helical, installed on these electrode devices at a right angle to the electrode surface, movable would present design problems. However, for an electrode device according to the invention, the fixation element can be advanced parallel to the electrode catheter even if the electrode surface is also parallel to the electrode catheter, since the flexibility of the electrode makes it possible to first affix the electrode with the fixation element in the correct position, e.g. with its free end, and then bend it in against tissue, e.g. the electrode thereafter being sutured at this position. It is also possible e.g. when a heart is to be stimulated between two electrodes, to devise the electrodes so they largely enclose the heart and to affix a fixation element to each one at one end, thereafter joining and linking the other ends at the electrode catheters, thereby affixing them to the heart with no need to suture the heart itself.

A further embodiment of the electrode device according to the invention is obtained when the electrode comprises a thin, flexible and electrically insulative electrode carrier on which at least one coiled conductor is arranged in a predetermined pattern to form the electrode surface and when a tubular element is attached to the electrode carrier as an extension of the electrode catheter, the fixation device thereby located at the free end of the tubular element.

The tubular element enables the stylet to stabilize the flexible electrode during introduction with no risk of damage to any other tissue, something which would otherwise be a risk if the stylet were completely unshielded. In addition, the stylet acts directly on the fixation device. Placement of the fixation device at the end of the tubular element affixes the most distal end of the electrode to tissue. This is an advantage when the fixation element is advanced parallel to the electrode catheter, since the actual penetration of tissue is facilitated if the fixation element can also be pressed against tissue.

In this context, it would be an advantage if at least one further fixation device is located at the opposite end of the tubular element. Both ends can then be affixed to tissue with the aid of the stylet, especially since there is a shortage of space around the tissue to which the electrode is to be affixed. The entire fixation procedure can then be controlled from outside the patient's body.

A further refinement of the electrode device according to the invention is achieved when the fixation element consists of a screw, the screw's direction of movement forming an acute angle to the electrode plane.

This is an advantage, since the electrode is introduced parallel to the tissue. The acute angle must be large enough for the fixation element to seat in tissue when advanced but not so large as to render the stylet incapable, without additional components, of manipulating the fixation element.

The angle should preferably be less than 15° so as to minimize impact on tissue.

Alternately, the fixation element can consist of a hook which is eccentrically rotatable around an axis parallel to the plane of the electrode surface. Rotation of this hook around the axis with the stylet causes the hook to emerge from the plane of the electrode surface, thanks to the eccentric rotation, and into the tissue the electrode is pressing against.

The invention will now be described in greater detail with reference to three figures, whereby
**FIG. 1** illustrates a first embodiment of the electrode device according to the invention.
**FIG. 2** shows an enlarged view of the fixation device in FIG. 1.
**FIG. 3** shows an enlarged view of an alternative version of the fixation device.
**FIG. 4** illustrates a second embodiment of the electrode device.

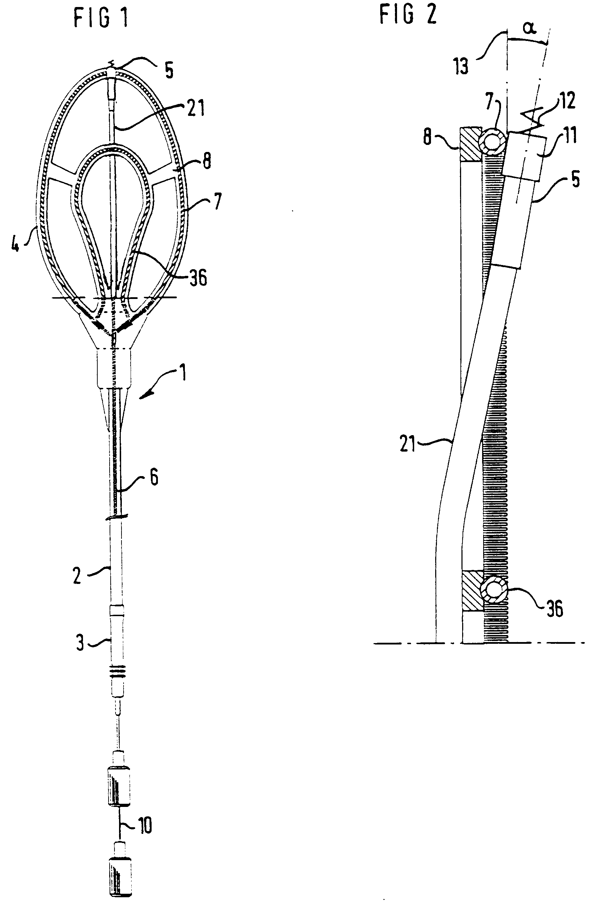

The electrode device 1 shown in FIG. 1 comprises an electrode catheter 2, a connection contact 3 , an electrode 4 and a fixation device 5. The connection contact 3, intended for coupling the electrode device 1 to a stimulation pulse generator (not shown) such as a defibrillator, is coupled to the electrode catheter 2 . A coiled electrode lead 6 passes from the connection contact 3 through the electrode catheter 2. Two coiled electrode conductors 7, 36 are coupled to the electrode lead 6 and arranged in a pattern on a thin, flexible and electrically insulative electrode carrier 8 in the electrode 4 . The electrode catheter 2 has a through channel into which a stylet 10 can be introduced to control the fixation device 5 . There is an insulated, coiled electrode conductor 21 , arranged as an extension of the electrode lead 6 up to the fixation device 5 , on the electrode carrier 8. Here, the stylet 10 runs inside the insulated electrode conductor 21 . The insulated electrode conductor 21 can also be uninsulated so as to form a part of the active electrode surface.

As shown in FIG. 2, the fixation device 5 comprises a sleeve 11 and a screw 12 . The screw 12 is screwed in/out of the shell 11 with the stylet 10 . The screw's 12 direction of movement follows a line which forms an acute angle α with the plane 13 of the electrode surface. Here, the angle α is formed by the insulated electrode conductor 21, running atop the electrode carrier 5, when crossing the second electrode conductor 36 and under the plane 13 of the electrode surface when it reaches the first electrode conductor 7 . As a consequence of the angle α , the screw 12 emerges from the plane 13 of the electrode surface when screwed out, thereby seating itself e.g. in pericardium around a heart. Since only pericardium is utilized for attaching the electrode to the heart, there is no damage to epicardium. In addition, the pericardium remains generally intact, and its protection of the heart is uncompromised.

If the selected placement of the electrode 4 during implantation proves to be unsuitable, the screw 12 can simply be retracted back into the sleeve 11 with the stylet 10 . The electrode 4 can then be moved to another part of the heart and affixed there.

Depending on the tissue to be stimulated and where the electrode device is to be situated to maximize stimulation efficacy, the angle α and the length of the screw 12 can vary. If tissue to be stimulated is sensitive and there is less sensitive tissue nearby, the electrode device 1 can be seated in the less sensitive tissue. Placement of the fixation device 5 does not have to be at the free end of the further electrode conductor 21.

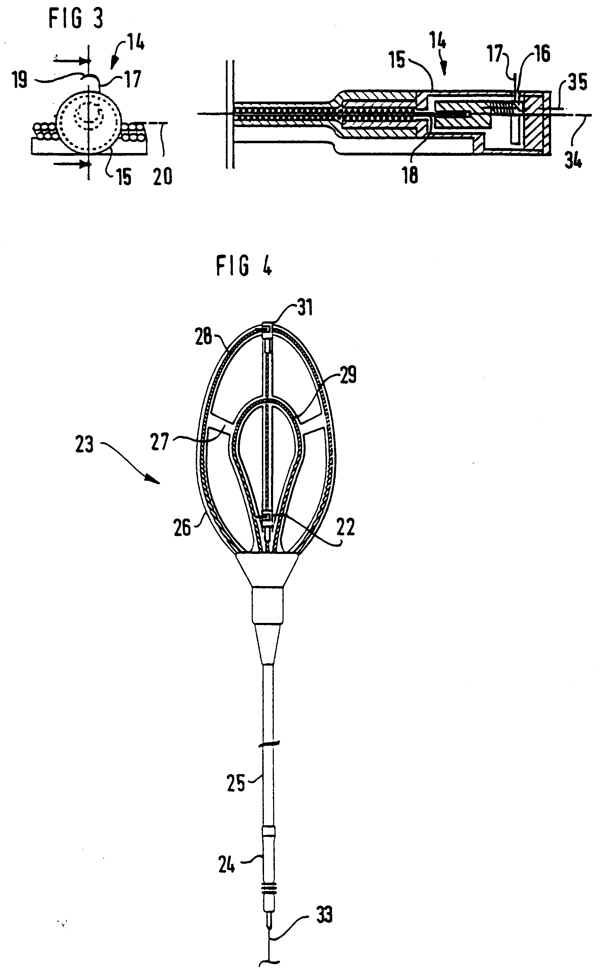

FIG. 3 illustrates an alternative fixation device 14 comprising a sleeve 15 with a groove 16 in the sleeve surface and an eccentrically rotatable hook 17 which is installed around the shell's 15 center axis 34 . The rotation axis 35 of the hook 17 is displaced in relation to the shell's 15 center axis 34 to produce the eccentricity. When the hook 17 is rotated by the action of a stylet 18, the pointed end 19 of the hook 17 advances down below the plane 20 of the electrode surface (dashed hook) and into underlying tissue, e.g. pericardium around the heart. The hook 17 can be simply retracted back into the sleeve 15 if the electrode 4 needs to be relocated.

Like the fixation device 5 in FIG. 2, the fixation device 14 shown in FIG. 3 can be placed anywhere along the electrode's 4 longitudinal axis. The shell 15 and the hook 17 can be designed so the hook 17 affixes the electrode 4 to tissue on the electrode's passive side.

The electrode device 23 in FIG. 4 is a second embodiment. In principle, the electrode device 23 is constructed in the same way as the electrode device 1 in FIG. 1 with a connection contact 24 for coupling the electrode device 23 to a stimulation pulse generator (not shown) such as a defibrillator, an electrode catheter 25 coupled at one end to the connection contact 24 and to an electrode 26 at the other end. The electrode 26 has a thin, flexible electrode carrier 27 on which a first coiled conductor 28 is arranged along the electrode carrier's 27 periphery and a second coiled conductor 29 is looped inside the first conductor 28 . An electrode lead (not shown) is provided inside the electrode catheter 25 to electrically couple the connection contact 24 to the first and the second conductors 28, 29 in the same way as shown in FIG. 1. A first fixation device 31 is located at the free end of the electrode carrier 27 , and a second fixation device 22 is located at its opposite end. A tubular element 32 is provided between the two fixation devices 22, 31. The two fixation devices 22, 31 are constructed as shown in FIG. 3 and manipulated with a stylet 33. With the two fixation devices 22, 31, the two ends of the electrode 26 can be affixed with an optional orientation to the tissue which is to be stimulated. Surgery can thereby be minimized, since the procedure for affixing the electrode to tissue is controlled from outside the patient's body.

## Claims

1. An electrode device (1) for a medical apparatus for stimulating with electrical pulses of a physiological function in a living creature, comprising an electrode catheter (2) in which an electrode lead (6) runs, an electrode (4) which is electrically coupled to the electrode lead (6) and a fixation device (5,14) on the electrode (4) for attaching the electrode (4) to tissue in the living creature, the fixation device (5,14) comprising a movable, fixation element (12,17) and the electrode catheter (2) having a channel to hold a stylet (10,18) which controls the fixation element (12,17), making the fixation element (12,17) controllable so it can be advanced out from the electrode surface against said tissue in order to affix the electrode (4) thereto, characterised in that the electrode (4) is thin and flexible, the electrode extends substantially in a single plane, and the electrode surface is spread out in said single plane.

2. An electrode device of claim 1, wherein the electrode (4) comprises a thin, flexible and electrically insulative electrode carrier (8) on which at least one coiled conductor (7) is arranged in a predetermined pattern to form the electrode surface and a tubular element (21) is attached to the electrode carrier (8) as an extension of the electrode catheter (2), the fixation device (5, 14) thereby located at the free end of the tubular element (21).

3. An electrode device of claim 2, wherein at least one further fixation device (22) is provided at the opposite end of the tubular element (21).

4. An electrode device of any of the above claims, wherein the fixation element consists of a screw (12), the movement direction of the screw (12) forming an acute angle (α) to the plane (13) of the electrode.

5. An electrode device of claim 4, wherein the angle (α) is less than 15°.

6. An electrode device of any of claims 1 to 3, wherein, the fixation element consists of a hook (17), the hook eccentrically rotatable around an axis which is parallel to the plane (20) of the electrode surface.

## Patentansprüche

1. Eine Elektrodenvorrichtung (1) für ein medizinisches Gerät zur Stimulation einer physiologischen Funktion in einem Lebewesen mit elektrischen Impulsen mit einem Elektrodenkatheter (2), in dem ein Leitung (6) verläuft, einer Elektrode (4), die elektrische mit der Leitung (6) verbunden ist, und einer Befestigungsvorrichtung (5, 14) an der Elektrode (4) zum Befestigen der Elektrode (4) an Gewebe in dem Lebewesen, wobei die Befestigungsvorrichtung (5, 14) ein bewegliches Befestigungselement (12, 17) aufweist und der Elektrodenkatheter (2) einen Kanal für einen Mandrin (10, 18), der das Befestigungselement (12, 17) steuert, hat, wodurch das Befestigungselement (12, 17) steuerbar wird, so daß es aus der Elektrodenoberfläche gegen das Gewebe herausbewegt werden kann, um die Elektrode (4) daran zu befestigen, **dadurch gekennzeichnet,** daß die Elektrode (4) dünn und flexibel ist, sich im wesentlichen in einer einzigen Ebene ausdehnt und die Elektrodenoberfläche über diese einzige Ebene verteilt ist.

2. Eine Elektrodenvorrichtung nach Anspruch 1, bei der die Elektrode (4) einen dünnen, flexiblen und elektrisch isolierten Elektrodenträger (8) aufweist, auf dem zumindest ein gewickelter Leiter (7) in einem vorbestimmten Muster angeordnet ist, um die Elektrodenoberfläche zu bilden, und ein rohrförmiges Element (21) als eine Verlängerung des Elektrodenkatheters (2) mit dem Elektrodenträger (8) verbunden ist, wodurch die Befestigungsvorrichtung (5, 14) am freien Ende des rohrförmigen Elementes (21) angeordnet ist.

3. Eine Elektrodenvorrichtung nach Anspruch 2, bei der zumindest eine weitere Befestigungsvorrichtung (22) am entgegengesetzten Ende des rohrförmigen Elementes (21) vorgesehen ist.

4. Eine Elektrodenvorrichtung nach einem der vorhergehenden Ansprüche, bei der das Befestigungselement aus einer Schraube (12) besteht und die Bewegungsrichtung der Schraube (12) einen spitzen Winkel (α) mit der Ebene (13) der Elektrode bildet.

5. Eine Elektrodenvorrichtung nach Anspruch 4, bei der der Winkel (α) kleiner als 15° ist.

6. Eine Elektrodenvorrichtung nach einem der Ansprüche 1 bis 3, bei der das Befestigungselement aus einem Haken (17) besteht, wobei der Haken exzentrisch um die Achse, die parallel zu der Ebene (20) der Elektrodenoberfläche ist, rotierbar ist.

## Revendications

1. Dispositif (1) formant électrode pour un dispositif médical destiné à stimuler par des impulsions électriques une fonction physiologique dans une créature vivante, comportant un cathéter (2) d'électrode dans lequel un conducteur (6) d'électrode s'étend, une électrode (4) qui est couplée électriquement au conducteur (6) d'électrode et un dispositif (5,14) de fixation sur l'électrode (4) destiné à fixer l'électrode (4) à des tissus dans la créature vivante, le dispositif (5,14) de fixation comportant un élément (12,17) mobile de fixation et le cathéter (2) d'électrode ayant un canal pour maintenir un stylet (10,18) qui commande l'élément (12,17) de fixation, rendant l'élément (12,17) de fixation commandable de sorte qu'il peut être avancé hors de la surface d'électrode contre le tissu, afin de fixer l'électrode (4) à celui-ci, caractérisé en ce que l'électrode (4) est mince et souple, l'électrode s'étend sensiblement dans un plan unique, et la surface d'électrode est répartie dans ledit plan unique.

2. Dispositif formant électrode suivant la revendication 1, dans lequel l'électrode (4) comporte un porteur (8) d'électrode isolant électriquement, mince et souple, sur lequel au moins un conducteur (7) enroulé est agencé suivant un motif déterminé à l'avance pour former la surface d'électrode et un élément (21) tubulaire est fixé au porteur (8) d'électrode en tant qu'un prolongement du cathéter (2) d'électrode, le dispositif (5,14) de fixation se trouvant ainsi à l'extrémité libre de l'élément (21) tubulaire.

3. Dispositif formant électrode suivant la revendication 2, dans lequel au moins un dispositif (22) de fixation supplémentaire est prévu à l'extrémité opposée de l'élément (21) tubulaire.

4. Dispositif formant électrode suivant l'une quelconque des revendications précédentes, dans lequel l'élément de fixation est constitué d'une vis (12), la direction de déplacement de la vis (12) formant un angle (α) aigu par rapport au plan (13) de l'électrode.

5. Dispositif formant électrode suivant la revendication 4, dans lequel l'angle (α) est inférieur à 15°.

6. Dispositif formant électrode suivant l'une quelconque des revendications 1 à 3, dans lequel l'élément de fixation est constitué d'un crochet (17), le crochet pouvant tourné de manière excentrique par rapport à un axe qui est parallèle au plan (20) de la surface d'électrode.
